(19) Europäisches Patentamt
European Patent Office
Office européen des brevets

(11) Veröffentlichungsnummer: **0 503 385 B1**

(12) **EUROPÄISCHE PATENTSCHRIFT**

(45) Veröffentlichungstag der Patentschrift: **09.08.95**

(51) Int. Cl.$^6$: **C09B 62/51**, C09B 62/09, C07C 317/28, //D06P1/38

(21) Anmeldenummer: **92103342.9**

(22) Anmeldetag: **27.02.92**

# (54) Verdoppelte Phenylazo- oder Naphthylazobenzole mit mehreren reaktiven Gruppen sowie deren Zwischenprodukte.

(30) Priorität: **09.03.91 DE 4107692**

(43) Veröffentlichungstag der Anmeldung: **16.09.92 Patentblatt 92/38**

(45) Bekanntmachung des Hinweises auf die Patenterteilung: **09.08.95 Patentblatt 95/32**

(84) Benannte Vertragsstaaten: **CH DE FR GB IT LI**

(56) Entgegenhaltungen:
**EP-A- 0 174 909**
**FR-A- 2 161 767**

(73) Patentinhaber: **BASF Aktiengesellschaft**
**Carl-Bosch-Strasse 38**
**D-67063 Ludwigshafen (DE)**

(72) Erfinder: **Siegel, Bernd, Dr.**
**Faberstrasse 39**
**W-6700 Ludwigshafen (DE)**
Erfinder: **Patsch, Manfred, Dr.**
**Fritz-Wendel-Strasse 4**
**W-6706 Wachenheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Reaktivfarbstoffe der Formel I

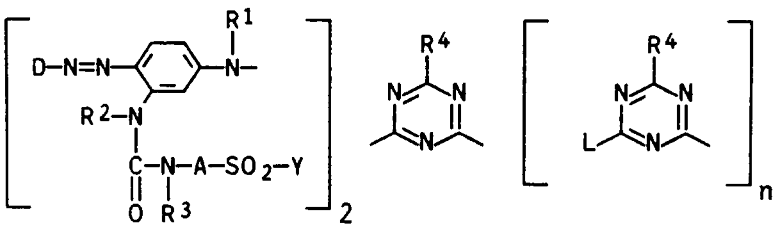

(I) ,

in der

- n — 0 oder 1,
- $R^1$, $R^2$ und $R^3$ — gleich oder verschieden sind und unabhängig voneinander jeweils Wasserstoff, $C_1$-$C_4$-Alkyl oder Phenyl,
- $R^4$ — Fluor, Chlor, Brom, $C_1$-$C_4$-Alkylsulfonyl, Phenylsulfonyl oder einen Rest der Formel

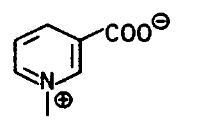

,

- L — ein Brückenglied,
- A — $C_2$-$C_8$-Alkylen, das gegebenenfalls durch 1 bis 3 Sauerstoffatome in Etherfunktion, Iminogruppen oder $C_1$-$C_4$-Alkyliminogruppen unterbrochen ist,
- Y — Vinyl oder einen Rest der Formel -$CH_2$-$CH_2$-Q, wobei Q für eine unter alkalischen Reaktionsbedingungen abspaltbare Gruppe steht, und
- D — Phenyl oder Naphthyl, wobei diese Reste ein- oder mehrfach durch Hydroxysulfonyl, Carboxyl, $C_1$-$C_4$-Alkoxycarbonyl, Cyano, $C_1$-$C_4$-Alkyl, $C_1$-$C_4$-Alkoxy, Halogen, Nitro oder Vinylsulfonyl substituiert sein können, bedeuten,

neue Phenylendiamine als deren Zwischenprodukte sowie die Verwendung der neuen Farbstoffe zum Färben oder Bedrucken von Hydroxygruppen oder Stickstoffatome aufweisenden Fasern.

Aus der EP-A-174 909 sind Reaktivfarbstoffe bekannt, die als reaktive Gruppe den Vinylsulfonylrest aufweisen, wobei dieser über eine Ureidogruppe an den Farbstoffrest geknüpft ist.

Weiterhin beschreibt die ältere Patentanmeldung EP-A-437 699 ähnliche Farbstoffe wie die eingangs genannten, jedoch sind diese nicht verdoppelt.

Aufgabe der vorliegenden Erfindung war es, neue Reaktivfarbstoffe bereitzustellen, die verdoppelt sind und eine Vinylsulfonylgruppe aufweisen, welche über eine Ureidogruppe an den Chromophor gebunden ist, wobei die neuen Farbstoffe günstige anwendungstechnische Eigenschaften aufweisen sollten.

Demgemäß wurden die eingangs näher bezeichneten Reaktivfarbstoffe der Formel I gefunden.

Die neuen Reaktivfarbstoffe der Formel I sind im vorliegenden Fall in der Regel in Form der freien Säure angegeben. Selbstverständlich werden jedoch auch ihre Salze mit umfaßt.

Als Salze kommen dabei Metall- oder Ammoniumsalze in Betracht. Metallsalze sind insbesondere die Lithium-, Natrium- oder Kaliumsalze. Unter Ammoniumsalzen im erfindungsgemäßen Sinne sind solche Salze zu verstehen, die entweder unsubstituierte oder substituierte Ammoniumkationen aufweisen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes $C_1$-$C_{20}$-Alkyl zu verstehen, das durch Hydroxylgruppen substituiert und/oder durch Sauerstoffatome unterbrochen sein kann.

Wenn in der obengenannten Formel I substituierte Phenylreste auftreten, so können dabei, sofern nicht anders vermerkt, z.B. $C_1$-$C_4$-Alkyl, $C_1$-$C_4$-Halogenalkyl, $C_1$-$C_4$-Alkoxy, Amino, $C_1$-$C_4$-Mono- oder Dialkylamino, Nitro, Formyl, Cyano, Carboxyl oder Hydroxysulfonyl als Substituenten in Betracht kommen. Sie weisen

in der Regel 1 bis 3 Substituenten auf.

Reste $R^1$, $R^2$ und $R^3$ sind z.B. Methyl, Ethyl, Propyl, Isopropyl, Butyl, Isobutyl oder sec-Butyl.

Reste $R^4$ sind z.B. Methylsulfonyl, Ethylsulfonyl, Propylsulfonyl, Isopropylsulfonyl oder Butylsulfonyl.

Reste A sind z.B. $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-CH(CH_3)-CH_2-$, $-CH(CH_3)-CH(CH_3)-$, $-(CH_2)_2-O-(CH_2)_2-$,$-(CH_2)_2-NH-(CH_2)_2-$, $-(CH_2)_2-N(CH_3)-(CH_2)_2-$, $-(CH_2)_2-O-(CH_2)_2-O-(CH_2)_2-$ oder $-(CH_2)_2-O-(CH_2)_2-O-(CH_2)_2-O-(CH_2)_2-$.

Y in Formel I steht u.a. für eine unter alkalischen Reaktionsbedingungen abspaltbare Gruppe. Solche Gruppen sind z.B. Chlor, $OSO_3H$, $SSO_3H$, $OP(O)(OH)_2$, $C_1$-$C_4$-Alkylsulfonyloxy, gegebenenfalls substituiertes Phenylsulfonyloxy, $C_1$-$C_4$-Alkanoyloxy, $C_1$-$C_4$-Dialkylamino,

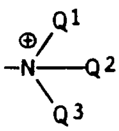 An$^{\ominus}$ , 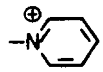 An$^{\ominus}$ , 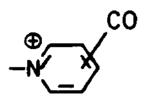 oder 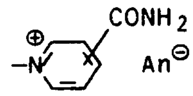 An$^{\ominus}$ ,

wobei $Q^1$, $Q^2$ und $Q^3$ gleich oder verschieden sind und unabhängig voneinander jeweils die Bedeutung von $C_1$-$C_4$-Alkyl oder Benzyl und $An^{\ominus}$ jeweils die Bedeutung eines Anions besitzen. (Geeignete Anionen sind z.B. Fluorid, Chlorid, Bromid, Iodid, Mono-, Di- oder Trichloracetat, Methylsulfonat, Phenylsulfonat oder 2- oder 4-Methylphenylsulfonat.)

Geeignete Brückenglieder L gehorchen z. B. der Formel

$-NH-L^1-NH-$ ,

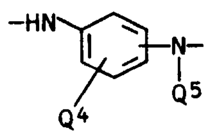 ,

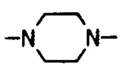 , 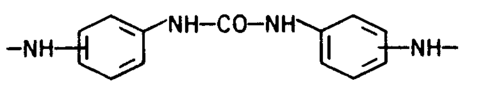 ,

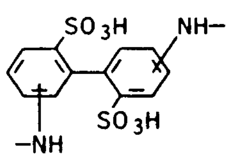 oder 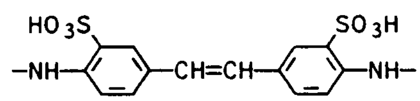 ,

wobei $L^1$ $C_2$-$C_8$-Alkylen, $Q^4$ Wasserstoff oder Hydroxysulfonyl und $Q^5$ Wasserstoff oder $C_1$-$C_4$-Alkyl bedeuten.

Einzelne Brückenglieder sind z. B. Reste der Formel

$-NH-C_2H_4-NH-$ , $-NH-C_3H_6-NH-$ ,

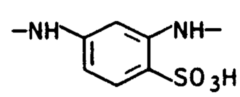 ,

oder 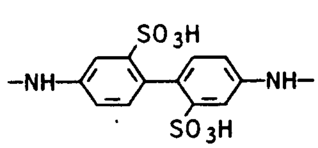 .

Reste D sind z.B. 2,4-Dihydroxysulfonylphenyl, 2,5-Dihydroxysulfonylphenyl, 2,5-Dihydroxysulfonyl-4-methylphenyl, 2,5-Dihydroxysulfonyl-6-chlorphenyl, 3,6,8-Trihydroxysulfonylnaphth-2-yl, 4,6,8-Trihydroxysulfonylnaphth-2-yl,1,5-Dihydroxysulfonylnaphth-2-yl oder 1,6-Dihydroxysulfonylnaphth-2-yl.

Bevorzugt sind Reaktivfarbstoffe der Formel I, in der

$R^1$, $R^2$ und $R^3$ jeweils Wasserstoff,

$R^4$ Fluor oder Chlor,

A $C_2$-$C_4$-Alkylen, das gegebenenfalls durch ein Sauerstoffatom in Etherfunktion unterbrochen ist, und

D Phenyl oder Naphthyl, wobei diese Reste durch Hydroxysulfonyl ein- bis dreifach substituiert sind, bedeuten.

Insbesondere bevorzugt sind Reaktivfarbstoffe der Formel I, in der

D Dihydroxysulfonylphenyl oder Trihydroxysulfonylnaphthyl bedeuten.

Insbesondere bevorzugt sind weiterhin Reaktivfarbstoffe der Formel I, in der, wenn n 1 bedeutet, L für einen Rest der Formel

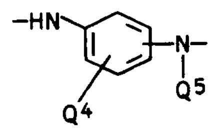

steht, in der $Q^4$ und $Q^5$ jeweils die obengenannte Bedeutung besitzen.

Die neuen Reaktivfarbstoffe der Formel I werden in vorteilhafter Weise erhalten, wenn man auf an sich bekanntem Weg z.B. ein Amin der Formel III

$D\text{-}NH_2$ (III),

in der D die obengenannte Bedeutung besitzt, diazotiert und mit einem Phenylendiamin der Formel II

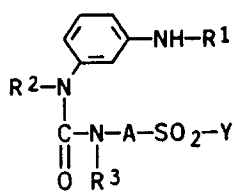

(II),

in der $R^1$, $R^2$, $R^3$, A und Y jeweils die obengenannte Bedeutung besitzen, kuppelt.

Der resultierende Azofarbstoff der Formel IV

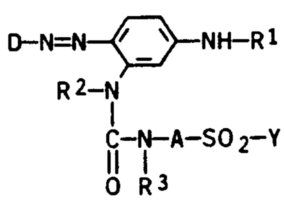

(IV),

in der D, $R^1$, $R^2$, $R^3$, A und Y jeweils die obengenannte Bedeutung besitzen, kann dann in bekannter Weise mit Trihalogentriazin umgesetzt werden. Die Umsetzung kann dabei im Eintopfverfahren oder mit Isolierung der Zwischenprodukte durchgeführt werden.

Um zu denjenigen Farbstoffen der Formel I zu gelangen, in der n O bedeutet, kann das Reaktionsprodukt mit weiterem Azofarbstoff IV umgesetzt werden.

Um zu denjenigen Farbstoffen der Formel I zu gelangen, in der n 1 bedeutet, kann das bei der Reaktion mit Trihalogentriazin entstehende Produkt mit einem Diamin der Formel V

$H_2N\text{-}L\text{-}NH_2$ (V),

in der L die obengenannte Bedeutung besitzt, umgesetzt werden.

Die neuen Reaktivfarbstoffe der Formel I eignen sich in vorteilhafter Weise zum Färben oder Bedrucken von Hydroxygruppen oder Stickstoffatome aufweisenden organischen Substraten. Solche Substrate sind beispielsweise Leder oder Fasermaterial, das überwiegend natürliche oder synthetische Polyamide oder natürliche oder regenerierte Cellulose enthält. Vorzugsweise eignen sich die neuen Farbstoffe zum Färben und Bedrucken von Textilmaterial auf der Basis von Baumwolle. Die erfindungsgemäßen Reaktivfarbstoffe können teilweise auch für den Ätzdruck verwendet werden.

Ein weiterer Gegenstand der vorliegenden Erfindung sind Phenylendiamine der Formel II

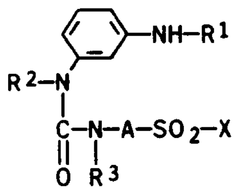

(II),

in der

| | |
|---|---|
| $R^1$, $R^2$ und $R^3$ | gleich oder verschieden sind und unabhängig voneinander jeweils Wasserstoff, $C_1$-$C_4$-Alkyl oder Phenyl, |
| A | $C_2$-$C_8$-Alkylen, das gegebenenfalls durch 1 bis 3 Sauerstoffatome in Etherfunktion, Iminogruppen oder $C_1$-$C_4$-Alkyliminogruppen unterbrochen ist, und |
| X | Vinyl, 2-Acetoxyethyl oder 2-Sulfatoethyl bedeuten. |

Was die beispielhafte Erläuterung der genannten Reste betrifft, sei auf die voranstehend vorgenommenen Ausführungen verwiesen.

Die neuen Phenylendiamine der Formel II können nach an sich bekannten Methoden erhalten werden. Beispielsweise indem man 3-Nitrophenylisocyanat der Formel VI

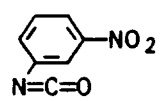

(VI)

mit einem schwefelhaltigen Amin der Formel VII

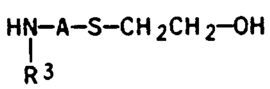

(VII),

in der $R^3$ und A jeweils die obengenannte Bedeutung besitzen, umsetzt und in der resultierenden Thioverbindung der Formel VIII

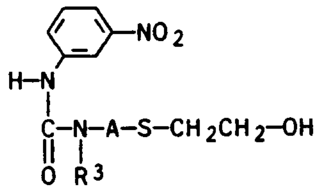

(VIII),

in der $R^3$ und A jeweils die obengenannte Bedeutung besitzen, auf an sich bekanntem Wege zunächst die Thiogruppierung zur Sulfongruppe oxidiert und anschließend die Nitrogruppe zur Aminogruppe reduziert.

Nach an sich bekannten Methoden (z. B. Veresterung mit Acetanhydrid, Acetylchlorid oder Schwefelsäure) kann dann die Hydroxyverbindung in die 2-Acetoxyethyl- oder 2-Sulfatoethylverbindung übergeführt werden, aus der wiederum auf üblichem Weg die Vinylverbindung erhalten werden kann.

Die erfindungsgemäßen Phenylendiaminderivate der Formel II sind wertvolle Zwischenprodukte für die Herstellung der neuen Reaktivfarbstoffe der Formel I.

Die folgenden Beispiele sollen die Erfindung näher erläutern.

Darstellung der Zwischenprodukte

Die in den folgenden Beispielen als Kupplungskomponente verwendeten Phenylendiamine können aus der in Beispiel 1 der älteren Patentanmeldung EP-A-437 699 beschriebenen Verbindungen wie folgt hergestellt werden.

Beispiel Z1

285 g (1 mol) der Verbindung der Formel

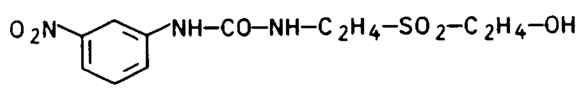

wurden mit 408 g (4 mol) Acetanhydrid auf 95 ° C erhitzt. Nach beendeter Umsetzung (DC-Kontrolle) wurde das überschüssige Acylierungsmittel mit Wasser zerstört und der sich bildende Niederschlag bei Raumtemperatur abgesaugt. Es wurden 323 g einer Verbindung der Formel

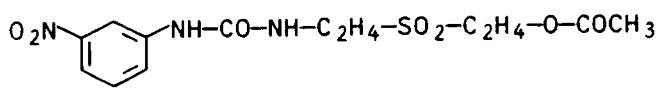

erhalten. Schmp.: 161 - 163 ° C
In analoger Weise können die folgenden Derivate hergestellt werden:

Bsp. Nr. Z2

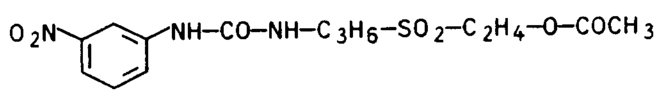

Bsp. Nr. Z3

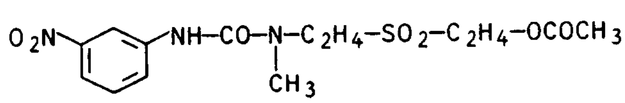

Beispiel Z4

359 g (1 mol) der in Beispiel Z1 beschriebenen Verbindung wurden in 1000 ml Methanol suspendiert und bei 60 ° C in Gegenwart von Raney-Nickel hydriert. Nach beendeter Wasserstoffaufnahme wurde vom Katalysator in der Wärme abfiltriert und der entstehende Niederschlag bei Raumtemperatur abgesaugt.
Es wurden 245 g der Verbindung der Formel

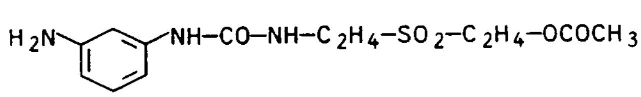

erhalten. Schmp.: 115 - 117 ° C

In analoger Weise können die folgenden Derivate hergestellt werden:

Bsp. Nr. Z5

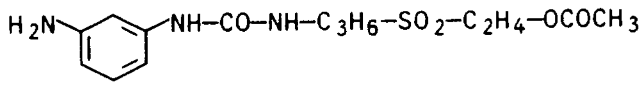

Bsp. Nr. Z6

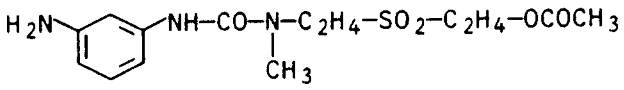

Beispiel Z7

285 g (1 mol) der in Beispiel Z1 verwendeten Ausgangsverbindung wurden in 1000 g Chlorsulfonsäure eingetragen und ca. 2 Stunden auf 30 bis 40 ° C erwärmt. Nach beendeter Veresterung wurde das Reaktionsgemisch, das den Schwefelsäureester der Formel

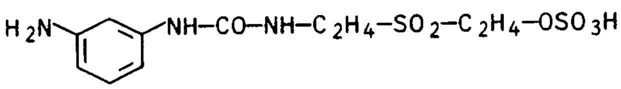

enthielt, mit Eiswasser zerlegt. Die resultierende Lösung wurde mit 20 gew.-%iger Natronlauge auf einen pH-Wert von 10 gestellt und bis zur vollständigen Eliminierung bei diesem pH-Wert gehalten. Der entstandene Niederschlag wurde abgesaugt, mit Wasser gewaschen und getrocknet. Es verblieben 190 g der Verbindung

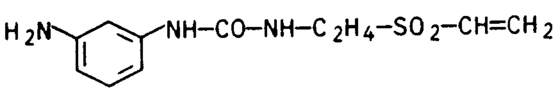

Schmp.: 141 - 142 ° C
In analoger Weise können folgende Derivate hergestellt werden:

Bsp. Nr. Z8

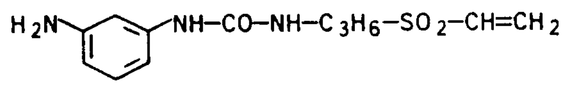

Bsp. Nr. Z9

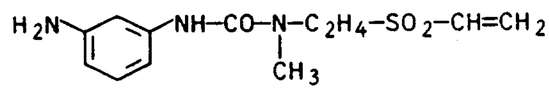

Darstellung der Farbstoffe

Beispiel 1

a) 101 g (0,4 mol) Anilin-2,4-disulfonsäure wurden in 400 ml Wasser salzsaurer diazotiert und zu einer Suspension aus 132 g (0,4 mol) das in Beispiel Z4 beschrieben Produkts in 500 ml Wasser gegeben. Die Kupplungsreaktion wurde bei 0 bis 5 °C und einem pH-Wert von 3 durch Zugabe von 2N Kaliumhydrogencarbonat-Lösung durchgeführt.
b) Die oben beschriebene Reaktionslösung wurde halbiert und die erste Hälfte mit 40,6 g (0,22 mol) Cyanurchlorid bei 0 bis 5 °C und einem pH-Wert von 5,5 kondensiert. Anschließend wurde die Lösung klärfiltriert und mit der zweiten Hälfte der Kupplungslösung bei 40 bis 50 °C und einem pH-Wert von 5,5 umgesetzt. Der resultierende Reaktivfarbstoff entspricht der Formel

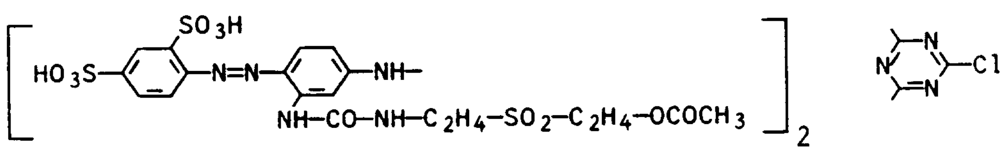

($\lambda_{max}$ = 378 nm)
und konnte durch Aussalzen isoliert werden. Er färbt Baumwolle in gelbem Ton mit sehr guter Lichtechtheit und sehr guten Naßechtheiten.
In analoger Weise können die in Tabelle 1 beschriebenen Farbstoffe der Formel

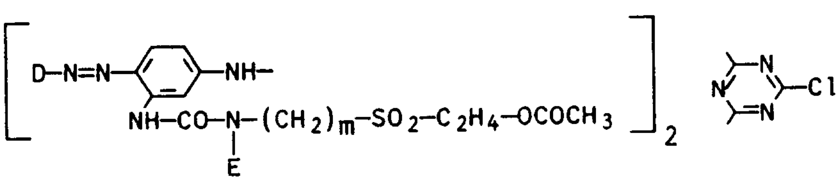

erhalten werden.
c) 254 g (1 mol) Anilin-2,4-disulfonsäure wurden in 1000 g Eis/Wasser-Gemisch bei 0 bis 5 °C mit 320 ml 23 gew.%iger Natriumnitritlösung diazotiert. Anschließend wurden 330 g (1,0 mol) des in Beispiel Z4 beschriebenen Produktes, in 500 g Wasser suspendiert, zugegeben und die Kupplungsreaktion mit Hilfe von 1 N Natriumhydrogencarbonatlösung bei einem pH-Wert von 3 durchgeführt. Die entstandene Kupplungslösung wurde daraufhin mit einem Gemisch aus 92 g (0,5 mol) Cyanurchlorid und 250 g Eiswasser versetzt. Dann wurde bei 0 bis 5 °C und einem pH-Wert von 5,5, der durch Zugabe von Natriumhydrogencarbonatlösung eingestellt wurde, der 1. Kondensationsschritt durchgeführt. Nach 1 Stunde wurde das Reaktionsgemisch für den 2. Kondensationsschritt auf 40 bis 45 °C erwärmt und ein

pH-Wert von 5,5 mittels Natriumhydrogencarbonatlösung eingehalten. Der resultierende Farbstoff entspricht als freie Säure der in Beispiel 1b beschriebenen Verbindung und wurde durch Sprühtrocknen isoliert.

Tabelle 1

| Bsp. Nr. | D | E | m | Farbton auf Baumwolle |
|---|---|---|---|---|
| 2 | 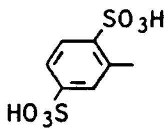 | H | 2 | gelb |
| 3 | 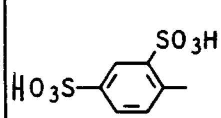 | H | 3 | gelb |
| 4 | 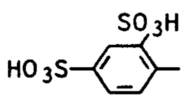 | $CH_3$ | 2 | gelb |
| 5 | 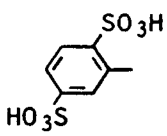 | H | 3 | gelb |
| 6 | 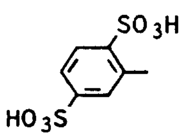 | $CH_3$ | 2 | gelb |
| 7 | 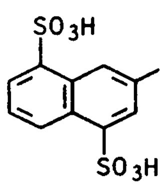 | H | 2 | gelb |
| 8 | 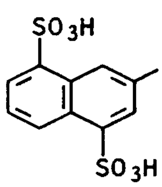 | H | 3 | gelb |

Tabelle 1 (Forts.)

| Bsp. Nr. | D | E | m | Farbton auf Baumwolle |
|---|---|---|---|---|
| 9 | 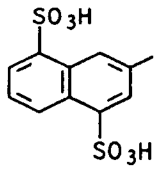 | $CH_3$ | 2 | gelb |
| 10 | 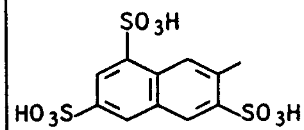 | H | 2 | goldgelb |
| 11 | 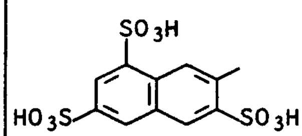 | H | 3 | goldgelb |
| 12 | 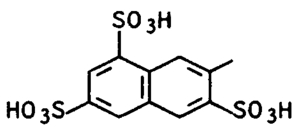 | $CH_3$ | 2 | goldgelb |
| 13 | 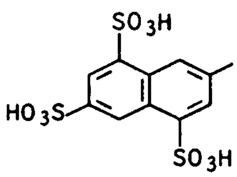 | $CH_3$ | 2 | goldgelb |
| 14 | 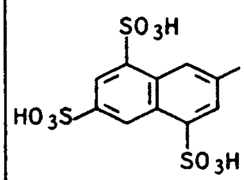 | H | 3 | goldgelb |
| 15 | 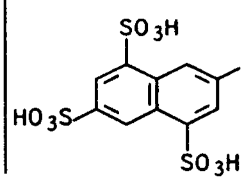 | $CH_3$ | 2 | goldgelb |

Tabelle 1 (Forts.)

| Bsp. Nr. | D | E | m | Farbton auf Baumwolle |
|---|---|---|---|---|
| 16 | 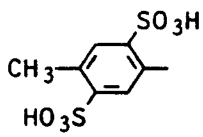 | H | 2 | gelb |
| 17 | 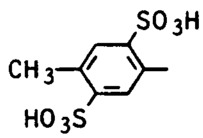 | H | 3 | gelb |
| 18 | 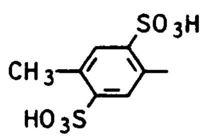 | $CH_3$ | 2 | gelb |
| 19 | 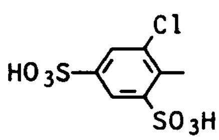 | H | 2 | gelb |
| 20 | 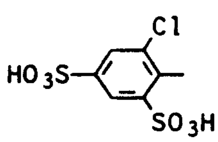 | H | 3 | gelb |
| 21 | 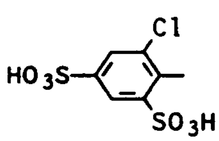 | $CH_3$ | 2 | gelb |

Beispiel 22

101 g (0,4 mol) Anilin-2,4-disulfonsäure wurden in 400 ml Wasser salzsauer diazotiert und zu einer Suspension aus 107,6 g (0,4 mol) des in Beispiel Z7 beschriebenen Produkts in 500 ml Wasser gegeben. Die Kupplungsreaktion wurde bei 0 bis 5°C durch Zugabe von 2N Kaliumhydrogencarbonat-Lösung durchgeführt. Anschließend wurde die Reaktionslösung halbiert, die erste Hälfte mit 40,6 g (0,22 mol) Cyanurchlorid versetzt und bei 0 bis 5°C mittels 2N Kaliumhydrogencarbonatlösung ein pH-Wert von 5,5 bis zur vollständigen Kondensation eingehalten. Die Reaktionslösung wurde klärfiltriert und mit der zweiten Hälfte der Kupplungslösung bei 40 bis 50°C und einem pH-Wert von 5,5 umgesetzt. Der resultierende Farbstoff konnte durch Eindampfen der Reaktionslösung isoliert werden. Er entspricht der Formel

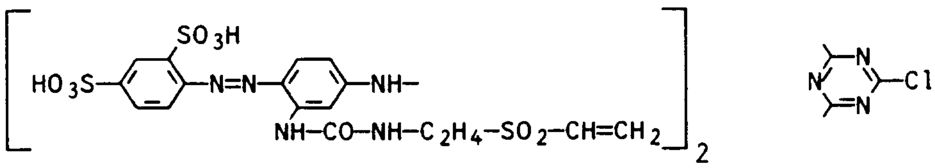

($\lambda_{max}$ = 375 nm)
und färbt Baumwolle in gelbem Ton.

In analoger Weise können die in Tabelle 2 aufgeführten Farbstoffe der Formel

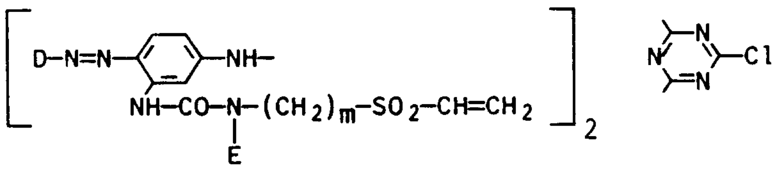

erhalten werden.

Tabelle 2

| Bsp. Nr. | D | E | m | Farbton auf Baumwolle |
|---|---|---|---|---|
| 23 | 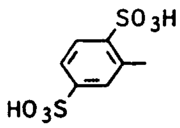 | H | 2 | gelb |
| 24 | 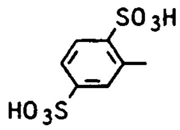 | H | 3 | gelb |
| 25 | 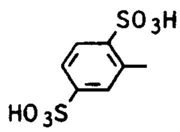 | $CH_3$ | 2 | gelb |
| 26 | 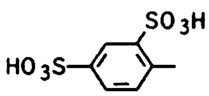 | H | 3 | gelb |
| 27 | 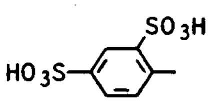 | $CH_3$ | 2 | gelb |
| 28 | 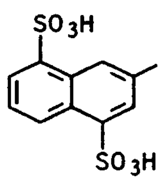 | H | 2 | gelb |

Tabelle 2 (Forts.)

| Bsp. Nr. | D | E | m | Farbton auf Baumwolle |
|---|---|---|---|---|
| 29 | 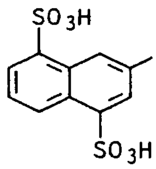 | H | 3 | gelb |
| 30 | 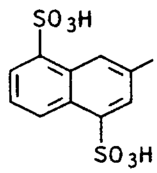 | $CH_3$ | 2 | gelb |
| 31 | 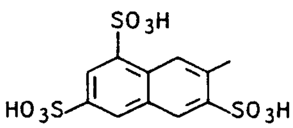 | H | 2 | goldgelb |
| 32 | 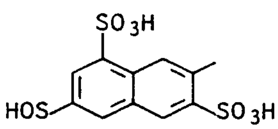 | H | 3 | goldgelb |
| 33 | 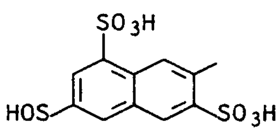 | $CH_3$ | 2 | goldgelb |
| 34 | 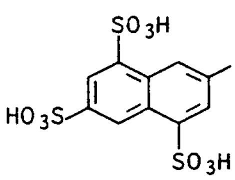 | H | 2 | goldgelb |

Tabelle 2 (Forts.)

| Bsp. Nr. | D | E | m | Farbton auf Baumwolle |
|---|---|---|---|---|
| 35 | 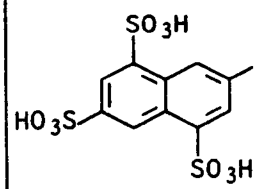 | H | 3 | goldgelb |
| 36 | 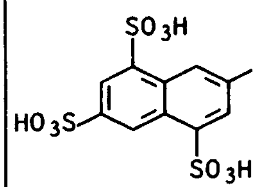 | $CH_3$ | 2 | goldgelb |
| 37 | 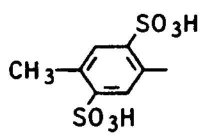 | H | 2 | gelb |
| 38 | 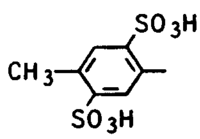 | H | 3 | gelb |
| 39 | 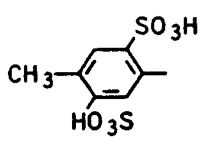 | $CH_3$ | 2 | gelb |
| 40 | 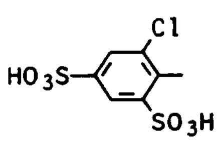 | H | 2 | gelb |

Tabelle 2 (Forts.)

| Bsp. Nr. | D | E | m | Farbton auf Baumwolle |
|---|---|---|---|---|
| 41 | 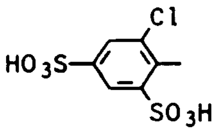 | H | 3 | gelb |
| 42 | 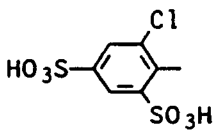 | $CH_3$ | 2 | gelb |

Beispiel 43

101 g (0,4 mol) Anilin-2,4-disulfonsäure wurden in 400 ml Wasser salzsauer diazotiert und zu einer Lösung aus 146,8 g (0,4 mol) des in Beispiel Z7 beschriebenen Schwefelsäureesters in 500 ml Wasser gegeben. Die Kupplungsreaktion wurde bei 0 bis 5 °C und einem pH-Wert von 3 durch Zugabe von 2N Natronlauge durchgeführt. Anschließend wurde die Kupplungslösung halbiert, die erste Hälfte mit 40,6 g (0,22 mol) Cyanurchlorid versetzt und bei 0 bis 5 °C ein pH-Wert von 5,5 mittels 2N Natronlauge eingestellt. Nach vollständiger Kondensation wurde die Reaktionslösung klärfiltriert und mit der zweiten Hälfte der Kupplungslösung bei 40 bis 50 °C und einem pH-Wert von 5,5 umgesetzt. Der entstandene Farbstoff konnte durch Aussalzen isoliert werden. Er entspricht der Formel

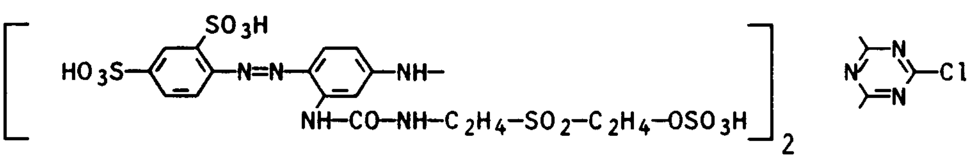

und färbt Baumwolle in gelbem Ton.

In analoger Weise können die in Tabelle 3 aufgeführten Farbstoffe der Formel erhalten werden

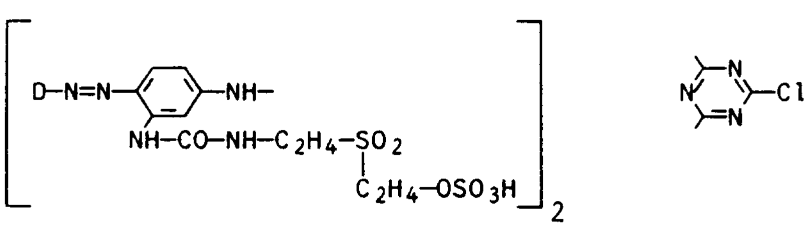

erhalten werden.

Tabelle 3

| Bsp. Nr. | D | Farbton auf Baumwolle |
|---|---|---|
| 44 | 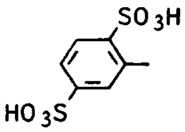 | gelb |
| 45 | 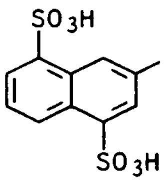 | gelb |
| 46 | 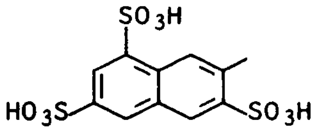 | gelb |
| 47 | 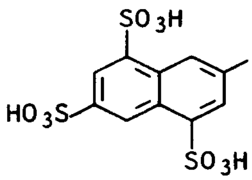 | gelb |

Tabelle 3 (Forts.)

| Bsp. Nr. | D | Farbton auf Baumwolle |
|---|---|---|
| 48 | 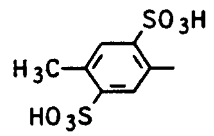 | gelb |
| 49 | 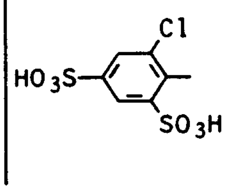 | gelb |

Beispiel 50

0,4 mol der in Beispiel 1 beschriebenen Kupplungslösung wurden mit 81,2 g (0,44 mol) Cyanurchlorid bei 0 bis 5 ° C und einem pH-Wert von 5,5 kondensiert. Die Reaktionslösung wurde anschließend klärfiltriert und mit 21,6 g (0,2 mol) para-Phenylendiamin versetzt. Zur vollständigen Umsetzung wurde das Reaktionsgemisch auf 40 bis 50 ° C erwärmt und durch Zugabe von 2N Natronlauge ein pH-Wert von 5,5 eingehalten. Der Farbstoff konnte durch Sprühtrocknen isoliert werden und entspricht der Formel

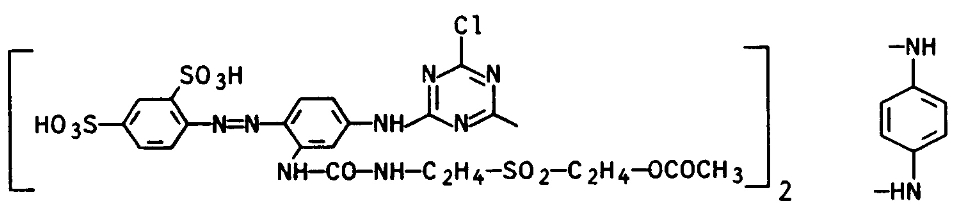

($\lambda_{max}$ = 391 nm)

Er färbt Baumwolle in gelbem Ton.

In analoger Weise können die in Tabelle 4 aufgeführten Farbstoffe der Formel

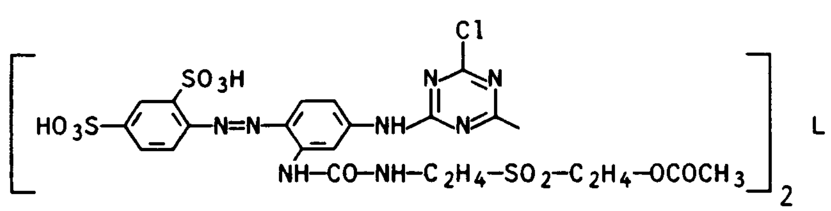

erhalten werden.

Tabelle 4

| Bsp. Nr. | L | Farbton auf Baumwolle |
|---|---|---|
| 51 | 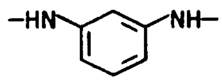 | gelb |
| 52 | $-HN-C_2H_4-NH-$ | gelb |
| 53 | $-HN-C_3H_6-NH-$ | gelb |
| 54 | 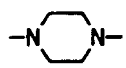 | gelb |
| 55 | 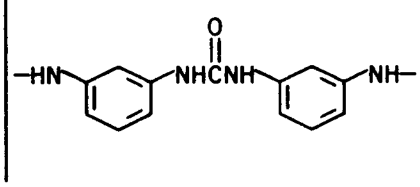 | gelb |

Beispiel 56

0,4 mol der in Beispiel 1 beschriebenen Kupplungslösung wurden mit 81,2 g (0,44 mol) Cyanurchlorid bei 0 bis 5 °C und einem pH-Wert von 5,5 umgesetzt. Die Reaktionslösung wurde anschließend klärfiltriert und mit 37,6 g (0,2 mol) 3,5-Diaminobenzolsulfonsäure versetzt. Zur vollständigen Umsetzung wurde das Reaktionsgemisch auf 40 bis 50 °C erwärmt und mit 2N Natronlauge ein pH-Wert von 5,5 eingehalten. Der resultierende Farbstoff wurde durch Eindampfen der Reaktionslösung isoliert. Er entspricht der Formel

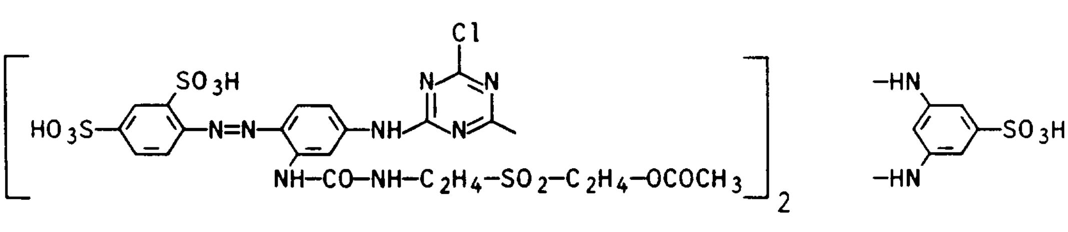

und färbt Baumwolle in gelbem Ton.

In analoger Weise können die in Tabelle 5 aufgeführten Farbstoffe der Formel

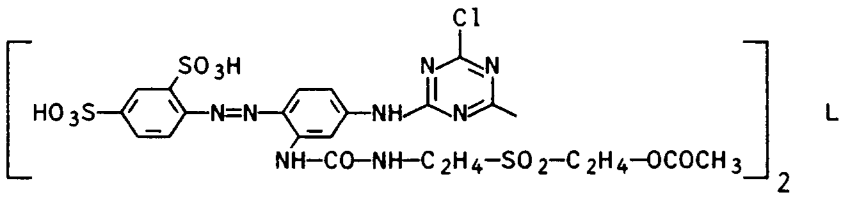

erhalten werden.

Tabelle 5

| Bsp. Nr. | L | Farbton auf Baumwolle |
|---|---|---|
| 57 | 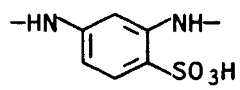 | gelb |
| 58 | 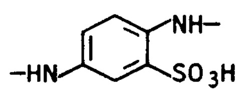 | gelb |
| 59 | 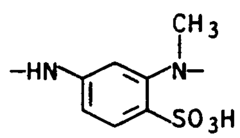 | gelb |
| 60 | 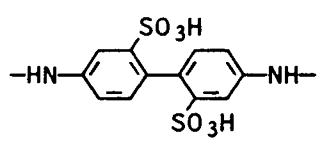 | gelb |
| 61 | 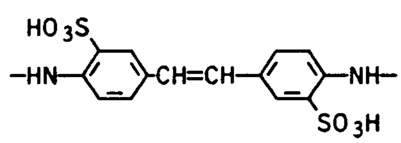 | gelb |

## Patentansprüche

**1.** Reaktivfarbstoffe der Formel I

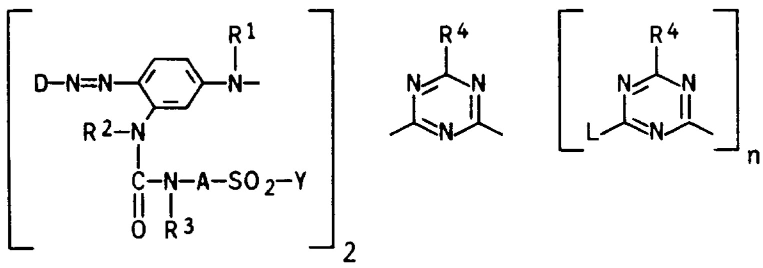

(I),

in der

n 0 oder 1

$R^1$, $R^2$ und $R^3$ gleich oder verschieden sind und unabhängig voneinander jeweils Wasserstoff, $C_1$-$C_4$-Alkyl oder Phenyl,

$R^4$ Fluor, Chlor, Brom, $C_1$-$C_4$-Alkylsulfonyl, Phenylsulfonyl oder einen Rest der Formel

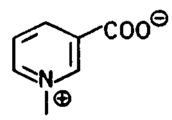

,

L ein Brückenglied,

A $C_2$-$C_8$-Alkylen, das gegebenenfalls durch 1 bis 3 Sauerstoffatome in Etherfunktion, Iminogruppen oder $C_1$-$C_4$-Alkyliminogruppen unterbrochen ist,

Y Vinyl oder einen Rest der Formel $-CH_2-CH_2-Q$, wobei Q für eine unter alkalischen Reaktionsbedingungen abspaltbare Gruppe steht, und

D Phenyl oder Naphthyl, wobei diese Reste ein- oder mehrfach durch Hydroxysulfonyl, Carboxyl, $C_1$-$C_4$-Alkoxycarbonyl, Cyano, $C_1$-$C_4$-Alkyl, $C_1$-$C_4$-Alkoxy, Halogen, Nitro oder Vinylsulfonyl substituiert sein können, bedeuten.

**2.** Reaktivfarbstoffe nach Anspruch 1, dadurch gekennzeichnet, daß

$R^1$, $R^2$ und $R^3$ jeweils Wasserstoff,

$R^4$ Fluor oder Chlor,

A $C_2$-$C_4$-Alkylen, das gegebenenfalls durch ein Sauerstoffatom in Etherfunktion unterbrochen ist, und

D Phenyl oder Naphthyl, wobei diese Reste durch Hydroxysulfonyl ein- bis dreifach substituiert sind, bedeuten.

**3.** Phenylendiamine der Formel II

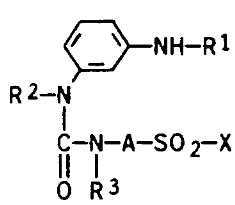

(II),

in der

$R^1$, $R^2$ und $R^3$ gleich oder verschieden sind und unabhängig voneinander jeweils Wasserstoff, $C_1$-$C_4$-Alkyl oder Phenyl,

A C$_2$-C$_8$-Alkylen, das gegebenenfalls durch 1 bis 3 Sauerstoffatome in Etherfunktion, Iminogruppen oder C$_1$-C$_4$-Alkyliminogruppen unterbrochen ist, und

X Vinyl, 2-Acetoxyethyl oder 2-Sulfatoethyl bedeuten.

**4.** Verwendung der Reaktivfarbstoffe gemäß Anspruch 1 zum Färben oder Bedrucken von Hydroxygruppen oder Stickstoffatome aufweisenden organischen Substraten.

**Claims**

**1.** A reactive dye of the formula I

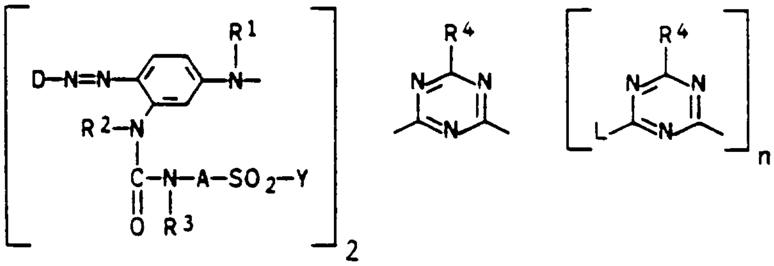

(I)

where

| | |
|---|---|
| n | is 0 or 1, |
| $R^1$, $R^2$ and $R^3$ | are identical or different and each is independently of the others hydrogen, $C_1$-$C_4$-alkyl or phenyl, |
| $R^4$ | is fluorine, chlorine, bromine, $C_1$-$C_4$-alkylsulfonyl, phenylsulfonyl or a radical of the formula |

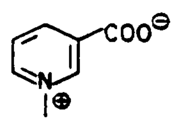

| | |
|---|---|
| L | is a bridge member, |
| A | is $C_2$-$C_8$-alkylene which may be interrupted by from 1 to 3 oxygen atoms in ether function, imino groups or $C_1$-$C_4$-alkylimino groups, |
| Y | is vinyl or a radical of the formula $-CH_2-CH_2-Q$, where Q is a group which is detachable under alkaline reaction conditions, and |
| D | is phenyl or naphthyl, which may each be monosubstituted or polysubstituted by hydroxysulfonyl, carboxyl, $C_1$-$C_4$-alkoxycarbonyl, cyano, $C_1$-$C_4$-alkyl, $C_1$-$C_4$-alkoxy, halogen, nitro or vinylsulfonyl. |

**2.** A reactive dye as claimed in claim 1, wherein

| | |
|---|---|
| $R^1$, $R^2$ and $R^3$ | are each hydrogen, |
| $R^4$ | is fluorine or chlorine, |
| A | is $C_2$-$C_4$-alkylene which may be interrupted by an oxygen atom in ether function, and |
| D | is phenyl or naphthyl, which may each be monosubstituted, disubstituted or trisubstituted by hydroxysulfonyl. |

3. A phenylenediamine of the formula II

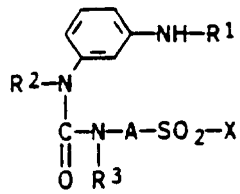

(II)

where

| | |
|---|---|
| $R^1$, $R^2$ and $R^3$ | are identical or different and each is independently of the others hydrogen, $C_1$-$C_4$-alkyl or phenyl, |
| A | is $C_2$-$C_8$-alkylene which may be interrupted by from 1 to 3 oxygen atoms in ether function, imino groups or $C_1$-$C_4$-alkylimino groups, and |
| X | is vinyl, 2-acetoxyethyl or 2-sulfatoethyl. |

4. The use of a reactive dye as claimed in claim 1 for dyeing or printing hydroxyl- or nitrogen-containing organic substrates.

**Revendications**

1. Colorants réactifs de formule I

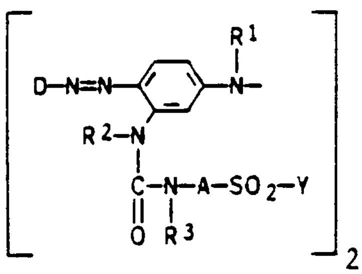 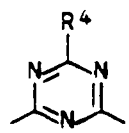 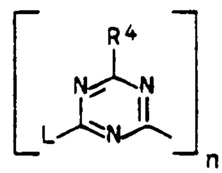

(I),

dans laquelle

| | |
|---|---|
| n | est mis pour 0 ou 1, |
| $R^1$, $R^2$ et $R^3$ | sont identiques ou différents et représentent chacun, indépendamment les uns des autres, un atome d'hydrogène ou un groupement alkyle en $C_1$-$C_4$ ou phényle, |
| $R^4$ | représente un atome de fluor, de chlore, de brome ou un groupement alkylsulfonyle en $C_1$-$C_4$, phénylsulfonyle ou un reste de formule |

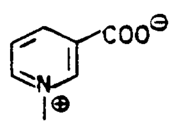

,

| | |
|---|---|
| L | est un chaînon de pontage, |
| A | représente un groupement alkylène en $C_2$-$C_8$ qui est éventuellement interrompu par 1 à 3 atomes d'oxygène en fonction éther, groupements imino ou groupements alkylimino en $C_1$-$C_4$, |
| Y | représente un groupement vinyle ou un reste de formule $-CH_2-CH_2-Q$, Q étant mis pour un groupement éliminable dans des conditions de réaction alcalines, et |
| D | représente un groupement phényle ou naphtyle, ces restes pouvant être substitués une ou plusieurs fois par des groupements hydroxysulfonyle, carboxyle, (alcoxy en $C_1$-$C_4$)carbonyle, cyano, alkyle en $C_1$-$C_4$, alcoxy en $C_1$-$C_4$, par des atomes d'halogène ou par des groupements nitro ou vinylsulfonyle. |

**2.** Colorants réactifs selon la revendication 1, caractérisés en ce que

$R^1$, $R^2$ et $R^3$ représentent chacun un atome d'hydrogène,

$R^4$ représente un atome de fluor ou de chlore,

A représente un groupement alkylène en $C_2$-$C_4$ qui est éventuellement interrompu par un atome d'oxygène en fonction éther, et

D représente un groupement phényle ou naphtyle, ces restes étant substitués une à trois fois par des groupements hydroxysulfonyle.

**3.** Phénylènediamines de formule II

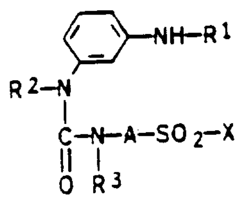

(II),

dans laquelle

$R^1$, $R^2$ et $R^3$ sont identiques ou différents et représentent chacun, indépendamment las uns des autres, un atome d'hydrogène ou un groupement alkyle an $C_1$-$C_4$ ou phényle,

A représente un groupement alkylène en $C_2$-$C_8$ qui est éventuellement interrompu par 1 à 3 atomes d'oxygène en fonction éther, groupements imino ou groupements alkylimino en $C_1$-$C_4$, et

X représente un groupement vinyle, 2-acétoxyéthyle ou 2-sulfatoéthyle.

**4.** Utilisation des colorants réactifs selon la revendication 1 pour la teinture ou l'impression de substrats organiques comportant des groupements hydroxy ou des atomes d'azote.